# EUROPÄISCHE PATENTANMELDUNG

(11) **EP 2 583 701 A1**
(43) Veröffentlichungstag der Anmeldung: **24.04.2013**
(21) Anmeldenummer: 11185966.6
(22) Anmeldetag: 20.10.2011
(51) Int. Cl.: A61M 1/36

(54) **Verfahren zum Beenden einer Hämodialyse**

(71) Anmelder: D_MED Consulting AG, 47800 Krefeld (DE)
(72) Erfinder: Breuch, Gerd, 53844 Troisdorf (DE); Biermann, Frank, 22455 Hamburg (DE); Yanagimoto, Yoji, 1030 Schaerbeek (BE)
(74) Vertreter: Patentanwälte ter Smitten Eberlein Rütten Partnerschaftsgesellschaft

(57) **Zusammenfassung**

Die Erfindung bezieht sich auf ein Verfahren zum Beenden einer Hämodialyse unter Verwendung eines Hämodialysegerätes (10). Bei dem Verfahren werden an den Patienten (50) angeschlossene arterielle und venöse Leitungen (42,44) mit einer Füllflüssigkeit befüllt, bis annähernd kein Patientenblut mehr in den Leitungen (42,44) enthalten ist. Dann werden die freien Enden der arteriellen und venösen Leitung (42,44) vom Patienten (50) abgenommen. Danach erfolgt ein Abpumpen der Füllflüssigkeit aus der arteriellen und venösen Leitung (42,44) durch Ultrafiltration durch die Dialysator-Membran (31) hindurch in die Dialysatkammer (29) des Dialysators (28) durch Betrieb der Saugpumpe (34), wobei sich die Leitungen (42,44) von den freien Enden aus mit Füllgas füllen. Sobald die Leitungen (42,44) mindestens größtenteils mit Füllgas gefüllt sind, werden die blutseitigen Leitungen (42,44) von dem Hämodialysegerät (10) entfernt.

## Beschreibung

Die Erfindung bezieht sich auf ein Verfahren zum Beenden einer Hämodialyse- Behandlung mit einem Hämodialysegerät.

Ein typisches Hämodialysegerät weist eine Dialysatseite und eine Blutseite auf, die durch eine Membran eines Dialysators voneinander getrennt sind. Zu der Dialysatseite zählen unter anderem eine Dialysatkammer dialysatseitig der Dialysator- Membran, eine Saugpumpe zwischen der Dialysatkammer und einem Abfalltank bzw. Abfallablauf, und ein Dialysatdruck- Sensor, der in der Regel zwischen der Dialysatkammer und dem Abfalltank bzw. -ablauf angeordnet ist. Zu der Blutseite zählen die Blutkammer des Dialysators, die der Dialysatkammer bezüglich der Dialysator- Membran fluidisch gegenüberliegt, sowie eine arterielle Leitung und eine venöse Leitung, die beide an die Blutkammer angeschlossen sind. Ferner ist eine Füllflüssigkeits-Quelle vorgesehen, die eine Füllflüssigkeit zum Füllen der blutseitigen Leitungen zur Verfügung stellt.

Aus DE 196 55 225 B4 ist bekannt, als Füllflüssigkeit Dialysat zu verwenden, das von einer Dialysatpumpe durch die Dialysator- Membran zur Blutseite und in die blutseitigen Leitungen gepumpt wird. Als Füllflüssigkeit kann aber auch eine Kochsalzlösung aus einem entsprechenden Vorratsbehälter dienen, die in die blutseitigen Leitungen gepumpt wird.

Beim Beenden der Hämodialyse- Behandlung, dem so genannten Ablegen, werden zunächst die beiden Leitungen der Blutseite, die den extrakorporalen Kreislauf bilden, mit der Füllflüssigkeit, gefüllt. Die beiden Leitungen werden dabei bevorzugt symmetrisch von dem Dialysator aus mit Füllflüssigkeit gefüllt. Die von dem Dialysator aus in die beiden Leitungen einfließende Füllflüssigkeit schiebt dabei das Patientenblut in den blutseitigen Leitungen vor sich her zurück in den Patienten. Auf diese Weise wird der Blutverlust des Patienten minimiert. Alternativ dann das Füllen mit Flüssigkeit auch durch Anschließen eines Füllflüssigkeitsvorrats an die arterielle Kanüle erfolgen, wobei die Blutpumpe die Füllflüssigkeit in einer Richtung durch beide Leitungen in Richtung venöser Leitung bzw. venöser Kanüle pumpt.

Schließlich wird bzw. werden die beiden blutseitige(n) Leitung(en) von dem Patienten abgenommen, so dass die beiden Kanülen der beiden Leitungen von den Blutgefäßen des Patienten entfernt sind. Die Leitungen und der Dialysator sind Einwegartikel, und kommen in den Haus- oder sogar Sondermüll. Die Haus- oder Sondermüll-Kosten können sich nach dem Gewicht des Mülls richten. In jedem Fall erschwert ein hohes Müllgewicht die Handhabung des Mülls.

Aufgabe der Erfindung ist es, das Müllgewicht für eine HämodialyseBehandlung zu verringern.

Diese Aufgabe wird erfindungsgemäß gelöst mit den Merkmalen des Anspruchs 1.

Gemäß dem erfindungsgemäßen Verfahren wird nach dem Abnehmen der freien Enden bzw. der Kanülen der arteriellen und der venösen Leitung von dem Patienten die Füllflüssigkeit aus der arteriellen Leitung und der venösen Leitung durch Ultrafiltration durch die Dialysator- Membran hindurch zurück in die Dialysatkammer des Dialysators ultrafiltriert. Dies erfolgt dadurch, dass die Saugpumpe die Füllflüssigkeit aus dem Dialysator abpumpt, während gleichzeitig ein entsprechend verringerter Strom oder ein Nullstrom an Dialysat auf der Dialysatseite in Richtung Dialysator gepumpt wird. Auf diese Weise wird auf der Dialysatseite der Membran ein Unterdruck generiert, wodurch die Füllflüssigkeit von der Blutseite durch die Dialysator -Membran ultrafiltriert wird. Da die Enden bzw. Kanülen der Leitungen von dem Patienten zuvor abgenommen wurden, füllen sich die Leitungen von den freien Enden und/oder von einem separaten Gaszugang und ggf. von Druckaufnehmeranschlüssenaus mit Füllgas, vorzugsweise mit Umgebungsluft.

Der Dialysator und die blutseitigen Leitungen sind nur für den Einmalgebrauch gedacht, sind also sogenannte Einwegartikel, und als solche Müll bzw. Sondermüll. Durch das Absaugen des Dialysats aus den Leitungen wird das müllrelevante Gewicht der Einwegartikel verringert, so dass ggf. die Müll-Kosten verringert werden und die Handhabung erleichtert wird.

Unter Hämodialyse ist vorliegend jede extrakorporale Blutbehandlung zu verstehen, beispielsweise auch die durch ein Akutgerät.

Vorzugsweise ist im Verlauf der venösen Leitung eine Luftfalle angeordnet und ist im Verlauf der arteriellen Leitung ein Leitungszugang vorgesehen, an dem das freie Ende bzw. die Küvette der venösen Leitung ankuppelbar ist. Der arterielle Leitungszugang dient darüber hinaus als Zugang für die Verabreichung von Medikamenten, beispielsweise Heparin, während der Dialyse, und ist daher standardmäßig häufig schon vorhanden.

Zunächst wird die Füllflüssigkeit aus der arteriellen Leitung abgepumpt, bis der Leitungs-Abschnitt distal des Leitungszugangs weitgehend mit Füllgas gefüllt und von der Füllflüssigkeit entleert ist. Daraufhin wird die arterielle Leitung distal des Leitungszugangs verschlossen, beispielsweise durch eine manuelle oder automatische Schlauchklemme oder durch Anhalten der im Verlauf der arteriellen Leitung angeordneten Blutpumpe, die beispielsweise als rotierende Peristaltikpumpe ausgebildet sein kann.

Die Luftfalle weist prinzipbedingt einen relativ großen Innenraum auf, aus dem die darin befindliche Füllflüssigkeit nicht vollständig von ihrem obenliegenden Flüssigkeitseinlass aus, der mit der Dialysatkammer des Dialysators verbunden ist, entleert werden kann. Der von der Luftfalle aus betrachtet distale, also patientenseitige, Abschnitt der venösen Leitung muss daher von dem freien Ende bzw. der Kanüle der venösen Leitung aus geleert werden.

Gleichzeitig mit oder nach dem Abpumpen der Füllflüssigkeit aus dem distalen Leitungsabschnitt der arteriellen Leitung wird die Füllflüssigkeit aus dem proximalen venösen Leitungsabschnitt zwischen dem Dialysator und der Luftfalle durch Ultrafiltration abgepumpt und mit dem nachfließenden Füllgas gefüllt. Hierzu muss gegebenenfalls ein entsprechender Gaszugang an der Luftfalle geöffnet werden, damit durch den Gaszugang Füllgas, beispielsweise Umgebungsluft, in den Leitungsabschnitt zwischen dem Dialysator und der Luftfalle von der Luftfalle aus nachfließen kann. Das Abpumpen der Füllflüssigkeit aus dem dialysatorseitigen Leitungsabschnitt der venösen Leitung wird beendet, sobald dieser Leitungsabschnitt weitgehend von der Füllflüssigkeit geleert ist. Der Bezugskörper für die Angaben "proximal" und "distal" ist vorliegend stets das Hämodialysegerät, nicht der Patient.

Anschließend wird das freie Ende bzw. die Kanüle der venösen Leitung an den Leitungszugang der arteriellen Leitung angekoppelt, beispielsweise durch Ankuppeln des Leitungsendes oder durch Einstechen der Kanüle in eine entsprechende Membran des Leitungszugangs. Dann wird die Füllflüssigkeit aus dem noch mit Füllflüssigkeit gefüllten Leitungsabschnitt distal der Luftfalle ultrafiltriert. Die Ultrafiltration erfolgt wieder dadurch, dass auf der Dialysatseite der Dialysator- Membran durch die Saugpumpe ein Unterdruck erzeugt wird, durch den die Füllflüssigkeit aus der Blutkammer durch die Dialysator- Membran hindurch in die Dialysatkammer abgesaugt wird. Auch hierbei wird gegebenenfalls der Gaszugang an der Luftfalle geöffnet, so dass Umgebungsluft hinter der Füllflüssigkeit nachfließen kann. Sobald auch der distale Leitungsabschnitt der venösen Leitung von der Füllflüssigkeit geleert und mit Füllgas gefüllt ist, wird die Saugpumpe und damit die Ultrafiltration gestoppt.

Vorzugsweise ist der arterielle Leitungszugang zwischen der Blutpumpe und dem Dialysator angeordnet. Hierdurch wird sichergestellt, dass die Blutpumpe zum Schließen der arteriellen Leitung vor dem Abpumpen der Füllflüssigkeit aus dem distalen Leitungsabschnitt der venösen Leitung benutzt werden kann. Ein separates manuelles Schließen der arteriellen Leitung, beispielsweise mit einer manuellen Schlauchklemme, ist nicht erforderlich. Alternativ kann der Leitungszugang auch distal der Blutpumpe vorgesehen sein. In diesem Fall kann die Blutpumpe an dem Abpumpen der Füllflüssigkeit aus der venösen Leitung mitwirken.

Gemäß einer bevorzugten Ausgestaltung weist das Hämodialysegerät einen Dialysatdruck- Sensor zwischen der Dialysatkammer des Dialysators und der Saugpumpe auf. Während der Dialysatentfernung wird ständig der Dialysatdruck durch den Dialysatdruck- Sensor bestimmt, wobei die Dialysatentfernung mit einem festgelegten Restvolumen volumengesteuert beendet wird, nachdem der Dialysatdruck eine festgelegte Luftankunfts-Druckschwelle durchschreitet, bevorzugt unterschreitet.

Der von Füllflüssigkeit an der Dialysator- Membran bewirkte Flusswiderstand ist erheblich kleiner als der von Füllgas bzw. Luft an der prinzipiell gasundurchlässigen Dialysator-Membran. Solange Füllflüssigkeit durch die Dialysator-Membran hindurch ultrafiltriert wird, ist der von dem Dialysatdruck-Sensor gemessene Druck zwischen der Dialysatkammer und der Saugpumpe erheblich höher als die festgelegte Luftankunfts-Druckschwelle. Sobald die Füllflüssigkeit annähernd vollständig aus den Leitungen und wenigstens teilweise aus der Blutkammer des Dialysators abgepumpt ist und mindestens teilweise Füllgas bzw. Luft an der Dialysator- Membran anliegt, fällt der von dem Dialysatdruck-Sensor gemessene Ansaug- Druck unter die festgelegte Luftankunfts-Druckschwelle. Dieses Ereignis ist ein sicheres Indiz dafür, dass die Füllflüssigkeit aus den Leitungen annähernd vollständig und aus der Dialysatkammer des Dialysators teilweise abgepumpt ist.

Sobald der von dem Dialysatdruck-Sensor gemessene Saugdruck die Luftankunfts-Druckschwelle durchschreitet, bzw. unterschreitet, wird eine volumengesteuerte Beendigung der Füllflüssigkeits-Entfernung eingeleitet. Dies bedeutet, dass nach der Unterschreitung der Luftankunfts-Druckschwelle nur noch ein festgelegtes Restvolumen aus der Dialysatkammer des Dialysators abgepumpt wird. Dieses Restvolumen kann Null betragen, kann auch größer als Null sein.

Gemäß einer bevorzugten Ausgestaltung erfolgt die Ultrafiltration bzw. das Abpumpen der Füllflüssigkeit aus den blutseitigen Leitungen primär volumengesteuert, wobei die Kontrolle des Dialysatdrucks durch den Dialysatdruck- Sensor nachgeordnet erfolgt. Grundsätzlich kann die Steuerung des Füllflüssigkeits-Abpumpens auch umgekehrt erfolgen, also primär druckgesteuert und sekundär, zur Sicherheit, auch volumengesteuert.

Vorzugsweise ist die Saugpumpe eine bidirektionale Ultrafiltrationspumpe, die zwischen der Dialysatkammer des Dialysators und einem Abfalltank oder Abfallauslass angeordnet ist. Ferner ist eine Dialysatpumpe fluidisch vor dem Dialysator angeordnet und zwangsweise mit einer Abfallpumpe, die Dialysat oder Füllflüssigkeit aus der Dialysatorkammer des Dialysators zu einer Bilanzierpumpe gekoppelt, beispielsweise mechanisch gekoppelt. Hierdurch wird mechanisch sichergestellt, dass die Dialysatpumpe jeweils exakt dieselbe Menge in Richtung Dialysator pumpt, wie die Abfallpumpe aus Richtung Dialysator abpumpt. Die separate Ultrafiltrationspumpe ist fluidisch parallel zu der Abfallpumpe angeordnet. Die Ultrafiltrationspumpe wird beim Füllen der Leitungen mit Dialysat im Gegenstrom betrieben, d.h. sie pumpt einen Teil der von der Abfallpumpe stromabwärts gepumpten Flüssigkeit über einen Bypass zurück zur Einlassseite der Abfallpumpe. Hierbei liegt die Pumprate der Ultrafiltrationspumpe stets unter der Pumprate der Abfallpumpe, damit nicht bereits verbrauchtes Dialysat rückwärts in den Dialysator gedrückt werden kann. Beim Füllflüssigkeits-Abpumpen bzw. bei der Dialysatentfernung wird die Ultrafiltrationspumpe im Parallelstrom betrieben, d.h. sie pumpt in der gleichen Richtung wie die Abfallpumpe. Im Parallelstrom betrieben bildet die Ultrafiltrationspumpe die Saugpumpe.

Vorzugsweise ist die Füllflüssigkeit Dialysat aus einer Dialysatquelle, wobei das Dialysat von einer Dialysatpumpe durch die Dialysator- Membran hindurch in die arterielle und die venöse Leitung gepumpt wird. Eine separate Füllflüssigkeit, beispielsweise eine Isotonische Kochsalzlösung, ist daher entbehrlich.

Gemäß einer bevorzugten Ausgestaltung ist an dem freien Ende der arteriellen Leitung eine arterielle Kanüle und an dem freien Ende der venösen Leitung eine venöse Kanüle vorgesehen. Nach dem Abnehmen der Leitungen einschließlich der Kanülen von dem Patienten, und vor dem Starten des Abpumpens bzw. der Ultrafiltration der Füllflüssigkeit werden die Kanülen von den Leitungs-Enden entfernt und entsorgt. Hierdurch wird das von den Kanülen ausgehende Verletzungsrisiko ausgeschlossen.

Im Folgenden wird ein Ausführungsbeispiel der Erfindung anhand der Zeichnungen näher erläutert:
Figur 1 zeigt schematisch ein Hämodialysegerät zur Durchführung einer Hämodialyse während des Füllens der blutseitigen Leitungen mit Füllflüssigkeit, und
Figur 2 zeigt schematisch das Hämodialysegerät der Figur 1 beim Abpumpen der Füllflüssigkeit aus den blutseitigen Leitungen.

In den Figuren ist schematisch ein Hämodialysegerät 10 dargestellt, das funktional in eine Dialysatseite 12 und eine Blutseite 14 unterteilt ist. Die Grenze zwischen der Dialysatseite 12 und der Blutseite 14 wird von einer Dialyse-Membran 31 in einem Dialysator 28 gebildet. Die Dialyse-Membran 31 trennt in dem Dialysator 28 eine Dialysatkammer 29 von einer Blutkammer 30.

Die Dialysatseite 12 weist eine Dialysatquelle 16 auf, die das Dialysat für die Dialyse zur Verfügung stellt. Die Dialysatquelle 16 wird von einem Dialysewasser- Tank 17 mit Dialysewasser 18 und einem Additiv-Tank 70 mit einem Dialysat- Additiv 72 gebildet. Dem Additiv-Tank 70 ist eine Additiv-Pumpe 74 nachgeordnet, die das Additiv 72 wohldosiert dem Dialysewasser-Strom zuführt. Es können auch mehrere Additiv-Tanks vorgesehen sein, wobei als Additiv insbesondere Elektrolyte und Puffersubstanzen vorgesehen sind. Das Dialysewasser kann, alternativ zu dem Dialysewasser-Tank 17, auch von einer (nicht dargestellten) Wasseraufbereitungs-Vorrichtung zur Verfügung gestellt werden, in der aus Trinkwasser aus dem öffentlichen Leitungsnetz das Dialysewasser hergestellt wird.

Bei dem weiter unten beschriebenen Füllen mit Füllflüssigkeit dient das Dialysat als Füllflüssigkeit, so dass die Dialysat- Quelle 16 in dem Fall auch eine Füllflüssigkeits- Quelle 16 bildet.

Im Verlauf der Leitung zwischen der Dialysatquelle 16 und der Dialysatpumpe 22 ist eine Dialysat- Durchlaufheizung 47 angeordnet, die das Dialysat bzw. die Füllflüssigkeit, derart erwärmt, dass es bzw. sie in dem Dialysator 28 ungefähr Körpertemperatur oder eine geringfügig oberhalb der Körpertemperatur liegende Temperatur hat. Vorliegend ist die Durchlaufheizung 47 in Strömungsrichtung vor dem Additiv₋Zulauf angeordnet, so dass die Durchlaufheizung 47 unmittelbar das Dialysewasser 18 bzw. die Füllflüssigkeit erwärmt, nicht jedoch das fertige Dialysat. Zwischen der Dialysat- Durchlaufheizung 47 und dem Dialysator 28 ist ein Temperatursensor 50 angeordnet. Ferner ist ein Temperaturregler 96 vorgesehen. Die Dialysat- Durchlaufheizung 47, der Temperatursensor 50 und der Temperaturregler 96 bilden zusammen einen Regelkreis zur Regelung der Füllflüssigkeits- bzw. Dialysat-Temperatur auf eine Solltemperatur. Die Solltemperatur ist so gewählt, dass während des Füllens die Füllflüssigkeit beim Erreichen des Patientenkörpers ungefähr Körpertemperatur hat.

Im weiteren Verlauf der Leitung zwischen der Dialysatpumpe 22 und dem Dialysator 28 ist ein Sterilisationsfilter 48 angeordnet, durch den das Dialysat durch Filterung derart "sterilisiert" wird, dass alle Viren, Bakterien, Endotoxine sowie Pilze von ihm zurückgehalten werden.

Im Verlauf der Leitung zwischen der Dialysatkammer 29 und der Abfallpumpe 24 ist ein Dialysat- Drucksensor 32 angeordnet, der den Druck der Dialysat- Flüssigkeit in diesem Leitungsabschnitt ermittelt. Parallel zu der Abfallpumpe 24 ist eine Ultrafiltrationspumpe 34 angeordnet, die in beiden Richtungen betrieben werden kann, also im Gegenstrom und im Parallelstrom zu der Abfallpumpe 24 betrieben werden kann. Im Parallelstrom betrieben arbeitet die Ultrafiltrationspumpe 34 als Saugpumpe 34.

Von der Dialysatkammer 29 des Dialysators 28 aus verläuft eine Leitung zu einer Abfallpumpe 24, die das Dialysat aus der Dialysatkammer 29 in einen Abfalltank 36 pumpt, in dem das verbrauchte Dialysat 38 gespeichert wird. Alternativ pumpt die Abfallpumpe 24 das Dialysat aus der Dialysatkammer 29 direkt in das öffentliche Abwassernetz.

Die Dialysatpumpe 22 und die Abfallpumpe 24 sind durch eine mechanische Verbindung 26 miteinander verbunden, so dass die Pumpraten der Dialysatpumpe 22 und der Abfallpumpe 24 stets absolut identisch sind. Auf diese Weise bilden die Dialysatpumpe 22 und die Abfallpumpe 24 eine sogenannte Bilanzierpumpe 20. Die mechanische Verbindung 26 kann beispielsweise dadurch realisiert sein, dass die Bilanzierpumpe 20 als Membranpumpe ausgebildet ist, wobei die eine Seite der Pumpenmembran die Membran der Dialysatpumpe 22 und die andere Seite der Pumpenmembran die Membran der Abfallpumpe 24 bildet,

Auf der Blutseite der Dialysator- Membran 31 befindet sich die Blutkammer 30 des Dialysators 28, in die eine arterielle Leitung 42 und eine venöse Leitung 44 münden. Im Verlauf der arteriellen Leitung 42 ist eine in beiden Richtungen betreibbare Blutpumpe 40 in Form einer peristaltischen Schlauchpumpe angeordnet, deren Pumprichtung und Pumprate von einer Gerätesteuerung gesteuert werden können. Die arterielle Leitung 42 weist an ihrem freien Ende eine arterielle Kanüle 43 und die venöse Leitung 44 an ihrem Ende eine venöse Kanüle 45 auf.

Die Kanülen 43,45 sind mit entsprechenden Kupplungen mit den jeweiligen Schlauch- bzw. Leitungsenden verbunden, so dass die Kanülen 43,45 für ihre Entsorgung bzw. zur Vermeidung von Verletzungen unmittelbar nach Abschluss der Behandlung sofort entfernt werden können. Die Kanülen 43,45 dienen der Verbindung der beiden Leitungen 42,44 mit entsprechenden Bfutgefäß-Zugängen bzw. Blutgefäßen des Patienten 50. Zwischen der arteriellen Kanüle 43 und der Blutpumpe 40 sind ein arterieller Drucksensor 52 und ein Luftsensor 54 angeordnet. Über den arteriellen Drucksensor 52 kann der Fluiddruck in der arteriellen Leitung 42 gemessen werden. Der Luftsensor 54 weist zum Einen einen optischen Luftblasendetektor auf, der Luftblasen in der vorbeifließenden Flüssigkeit innerhalb der arteriellen Leitung 42 detektieren kann, und weist zum Anderen eine von der Gerätesteuerung ansteuerbare Leitungsklemme auf, mit der bei Bedarf, und insbesondere bei Detektion von Luft, die arterielle Leitung 42 sofort vollständig geschlossen werden kann.

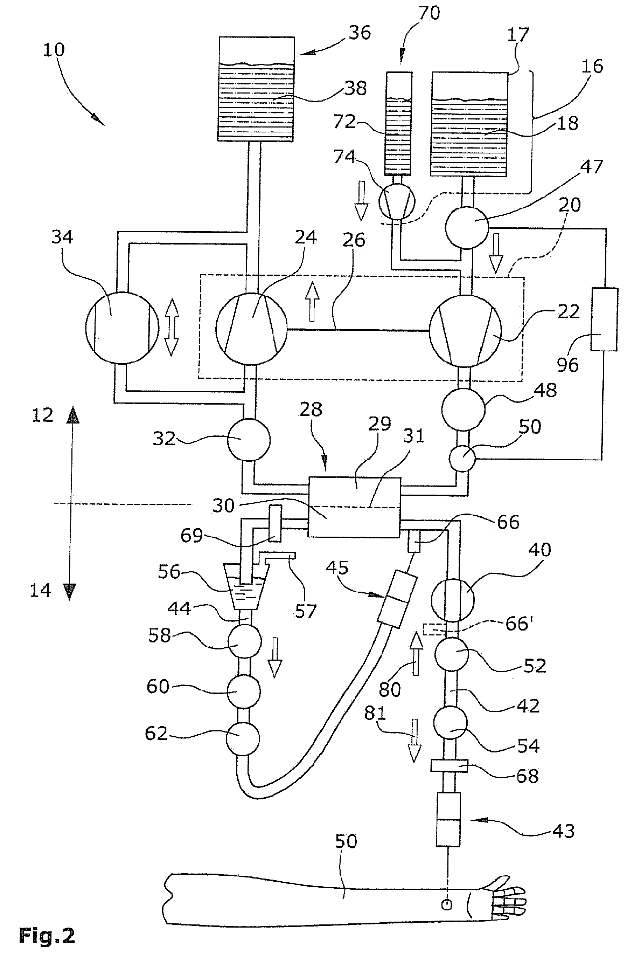

Zwischen dem Dialysator 28 und der Blutpumpe 40 weist die arterielle Leitung 42 einen Leitungszugang 66 auf, der zum Einen der optionalen Zugabe eines flüssigen Medikaments, beispielsweise Heparin, dient, und zum Anderen beim Abpumpen der Füllflüssigkeit aus den blutseitigen Leitungen 42,44 als Anschluss für das freie Ende der venösen Leitung 44 dient, wie in Figur 2 dargestellt. Alternativ ist der Leitungszugang 66' zwischen der Blutpumpe 40 und dem arteriellen Leitungsende bzw. der arteriellen Kanüle 43 angeordnet.

Im Verlauf der venösen Leitung 44 ist, von dem Dialysator 28 aus gesehen, zunächst einen Luftfalle 56 vorgesehen, in der Luftblasen der der hindurchfließenden Flüssigkeit zurückgehalten werden. Die Luftfalle 56 ist mit einem separaten Gaszugang 57 ausgestattet, der in das obere Ende des Innenraums der Luftfalle 56 mündet. Der Gaszugang 57 ist während der Hämodialyse und insbesondere auch beim Füllen der blutseitigen Leitungen 42,44 mit einem Pegelregler verbunden, der zusammen mit einer bidirektional betreibbaren Gaspumpe des Hämodialysegeräts 10 den Flüssigkeitspegel in dem Innenraum der Luftfalle 56 regelt. Ferner dient der Gaszugang 57 dazu, beim Abpumpen der Füllflüssigkeit aus der arteriellen Leitung 44 an der Rückseite der abfließenden Füllflüssigkeitssäule atmosphärischen Druck sicherzustellen, um das Abpumpen der Füllflüssigkeit überhaupt zu ermöglichen.

Zwischen der Luftfalle 56 und der venösen Kanüle 45 sind im Verlauf der venösen Leitung 44 ferner ein venöser Drucksensor 58, ein Luftsensor 60 sowie ein Farbsensor 62 angeordnet. Mit Hilfe des venösen Drucksensors 58 kann der Druck in der venösen Leitung 44 ermittelt werden. Der venöse Luftsensor 60 ist, analog zu dem arteriellen Luftsensor 54, ebenfalls mit einer ansteuerbaren Leitungsklemme ausgestattet. Der Farbsensor 62 ist möglichst nah an der Kanüle 45 angeordnet, und kann die Anwesenheit oder Nichtanwesenheit von Blut in der arteriellen Leitung 44 optisch detektieren. Der Farbsensor 62 fotometriert hierzu die transparente Leitung 44 bei einer bestimmten charakteristischen Wellenlänge.

Der Dialysator 28 und die beiden Leitungen 42,44 einschließlich der Kanülen 43,45 sind Wegwerfartikel, die nach einmaligem Gebrauch in den Haus- oder Sondermüll kommen.

Während der Hämodialyse pumpt die Blutpumpe 40 in Betriebsrichtung 80 Patientenblut aus dem Patientenkörper 50 durch die arterielle Leitung 42 in Richtung Dialysator 28, in dem das Patientenblut an der Dialysator-Membran 31 vorbei in die venöse Leitung 44 und zurück in den Patientenkörper 50 fließt. Gleichzeitig pumpt die Bilanzierpumpe 20 Dialysat durch die Dialysatkammer 29 des Dialysators 28. Wenn die eigentliche Hämodialyse, also der Vorgang der Blutwäsche, beendet ist, beginnt das sogenannte Ablegen, also die Trennungsvorbereitung und die Trennung des Hämodialysegeräts von dem Patienten. Danach können die desinfizierenden Maßnahmen und gegebenenfalls die Vorbereitungen für die folgende Hämodialyse- Behandlung des folgenden Patienten vorgenommen werden.

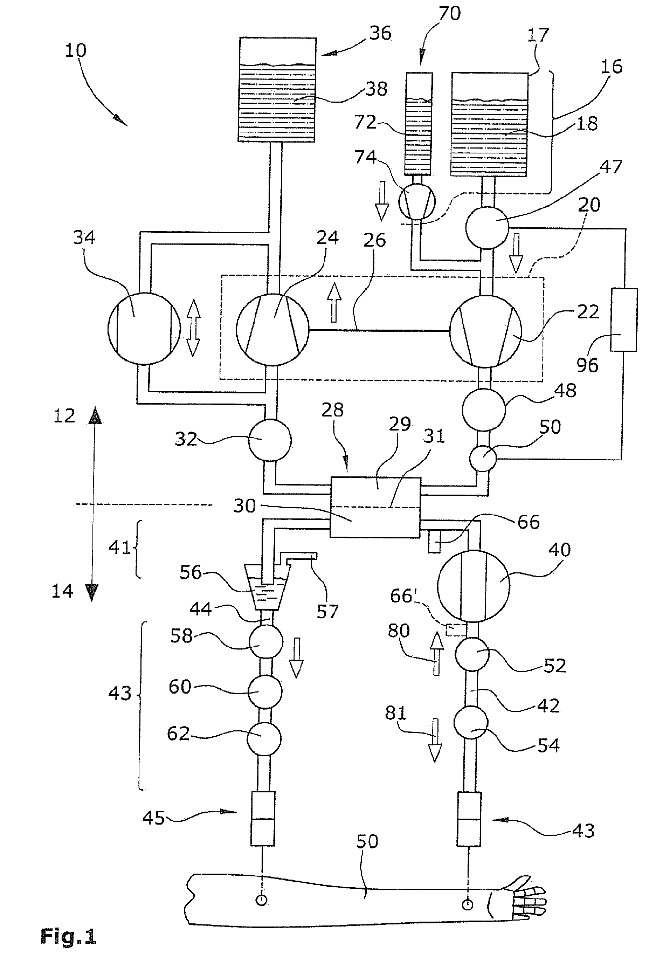

Die Trennungsvorbereitung beginnt mit dem Füllen der extrakorporalen Leitungen 42,44 mit einer Füllflüssigkeit, um auf diese Weise das Patientenblut möglichst vollständig aus den beiden Leitungen 42,44 zurück in den Patienten 50 zu pumpen. Als Füllflüssigkeit wird vorliegend Dialysat aus der Dialysatquelle 16 verwendet. Bei dieser Variante des Füllens der blutseitigen Leitungen 42,44 mit Füllflüssigkeit bleiben die beiden Kanülen 43,45 der beiden extrakorporalen Leitungen 42,44 direkt und unmittelbar an den Patienten 50 angeschlossen, wie in Figur 1 dargestellt.

Sobald das Füllen der Füllflüssigkeit von der Gerätesteuerung ausgelöst ist, werden zunächst die Blutkammer 30 des Dialysators 28, die arterielle Leitung 42 und die venöse Leitung 44 mit Füllflüssigkeit bzw. Dialysat aus der Füllflüssigkeits-Quelle 16 gefüllt, wodurch das Patientenblut aus beiden Leitungen 42,44 zurück in den Patienten 50 geschoben wird. Hierbei ist die Bilanzierpumpe 20 in Betrieb, so dass die Dialysatpumpe 22 Füllflüssigkeit bzw. Dialysat in Richtung Dialysator 28 pumpt, während gleichzeitig die Ultrafiltrationspumpe 34 im Gegenstrom arbeitet. Hierdurch wird die von der Füllflüssigkeits- Quelle 16 kommende und durch die Dialysatpumpe 22 gepumpte Füllflüssigkeit durch die Dialysator-Membran 31 hindurchgepresst, und zwar mit einer Durchtrittsrate, die mindestens der im Gegenstrom betriebenen Pumprate der Ultrafiltrationspumpe 34 entspricht. Hierbei wird die Blutpumpe 40 in Gegenrichtung 81 und mit ungefähr der halben Pumprate der Ultrafiltrationspumpe 34 betrieben. Die beiden blutseitigen Leitungen 42,44 werden so weit mit Füllflüssigkeit gefüllt, bis annähernd kein Patientenblut mehr in den Leitungen 42,44 vorhanden ist.

Neben der Volumensteuerung mit einem vorgegebenen Gesamtfüllvolumen wird das Füllen der Blutseite 14 mit Füllflüssigkeit durch den arteriellen Drucksensor 52 und den venösen Drucksensor 58 überwacht. Bei der Volumensteuerung wird grundsätzlich aus den aufaddierten Pumpzyklen der beteiligten Pumpe(n), vorliegend also aus den Pumpzyklen der Ultrafiltrationspumpe 34, auf das zur Blutseite gepumpte Füllflüssigkeits-Volumen geschlossen.

Sobald das Füllen der blutseitigen Leitungen 42,44 mit Füllflüssigkeit abgeschlossen ist, werden die arterielle und die venöse Leitung 42,44 von dem Patienten 50 abgenommen, werden also die beiden Kanülen 43,45 von den jeweiligen Zugängen bzw. den betreffenden Blutgefäßen des Patienten 50 getrennt. Die beiden Kanülen 43,45, die mit Kupplungen an den Leitungen 42,44 befestigt sind, werden von den Leitungen abgekoppelt und entsorgt, so dass von den Kanülen 43,45 keine Verletzungsgefahr mehr ausgeht.

Anschließend wird das Abpumpen der Füllflüssigkeit eingeleitet, durch das die Füllflüssigkeit, aus den Leitungen 42,44 schließlich weitgehend entfernt und durch Füllgas bzw. Umgebungsluft ersetzt wird. Beim Abpumpen der Füllflüssigkeit wird die Füllflüssigkeit aus der arteriellen und der venösen Leitung 42, 44 sowie teilweise aus der Dialysator- Blutkammer 30 abgepumpt bzw. ultrafiltriert. Bevor das Abpumpen beginnen kann, muss der Bediener an dem Hämodialysegerät 10 manuell bestätigen, dass alle Leitungen von dem Patienten abgenommen wurden.

Hierzu wird die Ultrafiltrationspumpe 34 in Parallelstrom betrieben, und wird auf diese Weise zu einer Saugpumpe 34, so dass durch die Abfallpumpe 24 und die Saugpumpe 34 in der Summe mehr Füllflüssigkeit bzw. Dialysat aus der Dialysatkammer 29 des Dialysators 28 abgepumpt wird als durch die Dialysatpumpe 22 zugepumpt wird. Die Mengendifferenz wird aus der Blutkammer 30 durch die Dialysator- Membran 31 hindurch in die Dialysatkammer 29 abgesaugt. Das Abpumpen der Füllflüssigkeit aus den blutseitigen Leitungen 42,44 kann in mehreren Phasen erfolgen.

In einer ersten Phase wird ausschließlich die arterielle Leitung 42 durch Abpumpen der Füllflüssigkeit entleert. Hierbei läuft die Blutpumpe 40 in Betriebsrichtung 80 mit derselben Pumprate wie die Saugpumpe 34. Die arterielle Leitung 42 wird hierbei so weit entleert, dass der Leitungsabschnitt zwischen dem Leitungszugang 66 bzw. dem alternativen Leitungszugang 66' und dem freien Ende der arteriellen Leitung praktisch frei von Flüssigkeit ist. Während des Abpumpens fließt vom freien Ende der arteriellen Leitung 42 als Füllgas Umgebungsluft in die arterielle Leitung 42.

Sobald die arterielle Leitung 42 ungefähr bis zum Leitungszugang 66 bzw. zum alternativen Leitungszugang 66' von Füllflüssigkeit entleert ist, wird das freie Ende der venösen Leitung 44 an den Leitungszugang 66 bzw. 66' angeschlossen, und wird anschließend in einer zweiten Phase der proximale Leitungsabschnitt 41 der venösen Leitung 44 entleert. Der proximale venöse Leitungsabschnitt 41 ist der Leitungsabschnitt zwischen der Luftfalle 56 und dem Dialysator 28. Beim Abpumpen der Füllflüssigkeit aus dem proximalen venösen Leitungsabschnitt 41 durch den Leitungszugang 66 steht die Blutpumpe 40 still. Ergänzend oder alternativ hierzu kann die arterielle Leitung 42 auch mit einer manuellen oder ggf. automatischen Schlauchklemme zwischen dem Leitungszugang 66 und dem freien Ende verschlossen werden. Auf diese Weise wird bei Betrieb der Saugpumpe 34 Füllflüssigkeit ausschließlich aus der venösen Leitung 44 abgepumpt. Ferner kann auch an dem freien Ende der venösen Leitung 44 diese mit einer manuellen Schlauchklemme verschlossen sein.

Wenn der Leitungszugang 66' zwischen der Blutpumpe 40 und dem freien Ende der arteriellen Leitung angeordnet ist, unterstützt die Blutpumpe das Abpumpen der Füllflüssigkeit aus der venösen Leitung 44.

Das Abpumpen der Füllflüssigkeit aus dem proximalen venösen Leitungsabschnitt 41 erfolgt volumengesteuert, bis der proximale Leitungsabschnitt 41 weitgehend oder vollständig von Füllflüssigkeit geleert ist. Der Gaszugang 57 ist beim Abpumpen in der zweiten Phase entweder zur Umgebungsluft geöffnet oder aber mit einer Füllgas-Pumpe des Hämodialysegeräts 10 verbunden, die Füllgas mit derselben Volumenrate in die Luftfalle 56 pumpt, wie von der Saugpumpe 34 Füllflüssigkeit abgepumpt wird.

Sobald die zweite Phase beendet ist, folgt die dritte Phase. Hierzu wird zunächst das freie Ende der venösen Leitung 44 an den Leitungszugang 66 angekoppelt. Ferner wird durch Anbringen einer manuellen Schlauchklemme in dem proximalen Leitungsabschnitt 41 auch in diesem Leitungsabschnitt der Fluidfluss unterbunden. Zwischen dem Leitungszugang 66 und dem freien Ende der arteriellen Leitung 42 bleibt der Fluidfluss ebenfalls unterbunden. Durch die Saugpumpe 34 wird nun über den Leitungszugang 66 aus dem distalen venösen Leitungsabschnitt 43 die Füllflüssigkeit abgepumpt und entsprechend mit Füllgas gefüllt. Das Füllgas fließt durch den Gaszugang 57 in Form von Umgebungsluft von der Luftfalle 56 aus in den distalen Leitungsabschnitt 43. Wenn der Gaszugang 57 an eine geräteseitige Füllgas-Pumpe angeschlossen ist, wird das Füllgas durch die Füllgas-Pumpe mit derselben Volumenrate in den Gaszugang 57 gepumpt, mit der die Saugpumpe 34 die Füllflüssigkeit aus dem distalen Leitungsabschnitt 43 abpumpt.

Statt in drei Phasen kann das Abpumpen der Füllflüssigkeit alternativ auch in einer einzigen Phase erfolgen, wobei hierbei unter Umständen in Kauf genommen werden muss, dass in der Luftfalle 56 ein Füllflüssigkeits-Restvolumen verbleibt, das nicht abgepumpt werden kann und dessen Volumen von der Innenkonstruktion der Luftfalle 56 abhängt. Beim einphasigen Füllflüssigkeits-Abpumpen wird zunächst das gesamte Füllgas aus der Luftfalle 56 über den Gaszugang 57 durch eine entsprechende geräteseitige Gaspumpe abgepumpt, die mit dem Gaszugang 57 fluidisch verbunden ist. Anschließend werden die beiden blutseitigen Leitungen 42,44 durch entsprechenden Betrieb der Saugpumpe 34 entleert, wobei der zeitliche Verlauf der Pumprate der Blutpumpe 40 darüber bestimmt, ob die beiden Leitungen 42,44 gleichzeitig, wechselweise oder nacheinander von der Füllflüssigkeit entleert werden. Ein Restvolumen an Füllflüssigkeit in der Luftfalle 56 kann nicht abgepumpt werden.

Nach der Beendigung des Abpumpens der Füllflüssigkeit aus den blutseitigen Leitungen 42,44 werden der Dialysator 28 und die beiden Leitungen 42,44 der Blutseite 14 von dem Hämodialysegerät 10 entfernt, wobei der nicht- fluidische Teil der Blutpumpe 40, die als Schlauchpumpe ausgebildet ist, an dem Hämodialysegerät 10 verbleibt. Das gleiche gilt für die Sensoren 52,54,58,60,62 aus denen lediglich die Messstrecke in Form der Leitungen 42,44, die als flexible und transparente Schläuche ausgebildet sind, entnommen werden. Der Dialysator 28 und die beiden Leitungen 42,44 der Blutseite 14 sind Einwegartikel, und müssen in der Regel als Sondermüll entsorgt werden.

## Patentansprüche

1. Verfahren zum Beenden einer Hämodialyse- Behandlung eines Patienten (50) mit einem Hämodialysegerät (10), wobei das Hämodialysegerät (10) aufweist:
eine Dialysatseite (12) mit einer Dialysatkammer (29) eines eine Membran (31) aufweisenden Dialysators (28) und einer Saugpumpe (34), die Flüssigkeit aus der Dialysatkammer (29) abpumpt,
eine Blutseite (14) mit einer arteriellen Leitung (42), einer Blutpumpe (40), einer Blutkammer (30) des Dialysators (28) und einer venösen Leitung (44), und
eine Füllflüssigkeits-Quelle (16), die eine Füllflüssigkeit zum Füllen der blutseitigen Leitungen (42,44) zur Verfügung stellt,
mit den Verfahrensschritten:
Füllen der an den Patienten (50) angeschlossenen arteriellen Leitung (42) und venösen Leitung (44) mit der Füllflüssigkeit, bis annähernd kein Patientenblut mehr in den Leitungen (42,44) enthalten ist,
Abnehmen der freien Enden der arteriellen und der venösen Leitung (42,44) von dem Patienten (50),
Abpumpen der Füllflüssigkeit aus der arteriellen und venösen Leitung (42,44) durch Ultrafiltration durch die Dialysators-Membran (31) hindurch in die Dialysatkammer (29) des Dialysators (28) durch Betrieb der Saugpumpe (34), wobei sich
die Leitungen (42,44) von den freien Enden aus mit Füllgas füllen, und
sobald die Leitungen (42,44) mindestens größtenteils mit Füllgas gefüllt sind: Entfernen der blutseitigen Leitungen (42,44) von dem Hämodialysegerät (10).

2. Verfahren zum Beenden einer Hämodialyse nach Anspruch 1, wobei die venöse Leitung (44) eine Luftfalle (56) und die arterielle Leitung (42) einen Leitungszugang (66; 66') aufweist, an den das freie Ende der venösen Leitung (44) ankuppelbar ist, mit den Verfahrensschritten beim Füllflüssigkeits-Abpumpen:
Abpumpen der Füllflüssigkeit aus der arteriellen Leitung (42),
Verschließen der arteriellen Leitung (42) distal des Leitungszugang (66; 66'),
Ankuppeln des freien Ende des der venösen Leitung (44) an den Leitungszugang (66, 66') der arteriellen Leitung (42), und
Abpumpen der Füllflüssigkeit aus der venösen Leitung (44) durch den arteriellen Leitungszugang (66).

3. Verfahren zum Beenden einer Hämodialyse nach Anspruch 2, wobei der arterielle Leitungszugang (66, 66') zwischen der Blutpumpe (40) und dem Dialysator (28) oder zwischen der Blutpumpe (40) und dem freien Ende der arteriellen Leitung (42) angeordnet ist.

4. Verfahren zum Beenden einer Hämodialyse nach einem der vorangegangenen Ansprüche, wobei ein Dialysatdruck-Sensor (32) zwischen der Dialysatkammer (29) und der Saugpumpe (34) vorgesehen ist, mit dem Verfahrensschritt:
während des Abpumpens der Füllflüssigkeit: ständige Bestimmung des Fluiddrucks durch den Dialysatdruck-Sensor (32), wobei das Füllflüssigkeits- Abpumpen volumengesteuert beendet wird, wenn der von dem Dialysatdruck-Sensor (32) gemessene Fluiddruck eine festgelegte Gasankunfts-Druckschwelle durchschreitet, und
bevorzugt unterschreitet.

5. Verfahren zum Beenden einer Hämodialyse nach einem der vorangegangenen Ansprüche, wobei am freien Ende der arteriellen Leitung (42) eine entfernbare arterielle Kanüle (43) und am freien Ende der venösen Leitung (44) eine venöse Kanüle (45) vorgesehen ist, mit dem Verfahrensschritt:
Nach dem Abnehmen der Leitungen (42,44) einschließlich der Kanülen (43,45) vom Patienten (50) und vor dem Füllflüssigkeits-Abpumpen:
Entfernen der Kanülen (43,45) von den Leitungs-Enden.

6. Verfahren zum Beenden einer Hämodialyse nach einem der vorangegangenen Ansprüche, wobei das Füllflüssigkeits- Abpumpen zwangsweise beendet wird, wenn ein vorgegebenes Soll-Gesamtvolumen aus den blutseitigen Leitungen (42,44) und dem Dialysator (28) abgepumpt wurde.

7. Verfahren zum Beenden einer Hämodialyse nach einem der vorangegangenen Ansprüche, wobei
die Saugpumpe (34) eine bidirektionale Ultrafiltrationspumpe (34) ist,
eine Dialysatpumpe (22) zwischen einer Dialysatquelle (16) und der Dialysatkammer (29) zwangsweise mit einer separaten
Abfallpumpe (24), die fluidisch parallel zu der Ultrafiltrationspumpe (34) angeordnet ist, zu einer Bilanzierpumpe (20) gekoppelt ist, und
die Ultrafiltrationspumpe (34) beim Füllen im Gegenstrom und beim Füllflüssigkeits- Abpumpen im Parallelstrom zu der Abfallpumpe (24) pumpt.

8. Verfahren zum Beenden einer Hämodialyse nach einem der vorangegangenen Ansprüche, wobei die Füllflüssigkeit Dialysat aus einer Dialysatquelle (16) ist, das von einer Dialysatpumpe (22) durch die Dialysator- Membran (31) in die arterielle und die venöse Leitung (42,44) gepumpt wird.

9. Verfahren zum Beenden einer Hämodialyse nach einem der vorangegangenen Ansprüche, wobei das Füllgas Umgebungsluft ist.
